# EUROPÄISCHE PATENTSCHRIFT

(11) **EP 0 654 470 B1**
(45) Veröffentlichungstag und Bekanntmachung des Hinweises auf die Patenterteilung: **03.01.2001**
(21) Anmeldenummer: 94116966.6
(22) Anmeldetag: 27.10.1994
(51) Int. Cl.: C07D 275/03, C07D 275/04, A01N 43/80

(54) **Lagerstabile wässrige Lösungen von Isothiazolin-3-onen**
Storage stable aqueous solutions of isothiazolen-3-ones
Solutions aqueuses d'isothiazolin-3-ones stables au stockage

(30) Priorität: 18.11.1993 DE 4339248
(43) Veröffentlichungstag der Anmeldung: 24.05.1995
(73) Patentinhaber: ETC C.V., Hamilton (BM)
(72) Erfinder: Lindner, Wolfgang, Dr., D-30926 Seelze (DE)
(74) Vertreter: Geissler, Bernhard, Dr. jur., Dipl.-Phys.

(56) Entgegenhaltungen:
- EP-A- 0 436 744
- US-A- 3 870 795
- US-A- 4 067 878

## Beschreibung

Die vorliegende Erfindung betrifft lagerstabile wäßrige Lösungen von Isothiazolin-3-onen, die ein Edelmetallion enthalten.

Isothiazolin-3-one sind mikrobiozid wirkende Substanzen, die wasserhaltigen technischen Materialien, wie beispielsweise Dispersionen, Dispersionsfarben, Klebstoffen, Gelatine, Leimen auf nativer Basis, Tensidlösungen oder funktionellen Flüssigkeiten wie Bohrlochspülungen und Metallbearbeitungsflüssigkeiten zum Schutz vor Mikroorganismen (Bakterien, Schimmelpilze, Hefen, Algen) zugesetzt werden können. Sie vereinen dabei hohe mikrobiologische Aktivität, leichte Abbaubarkeit in der Umwelt und leichte Dosierbarkeit. Konservierungsmittel auf Basis von Isothiazolin-3-onen werden beim Endverbraucher in Form von verdünnten Formulierungen eingesetzt, wobei als Verdünnungsmittel Wasser eingesetzt werden soll.

Allerdings sind Isothiazolin-3-one in wäßriger Lösung relativ instabil und werden durch Zersetzung leicht inaktiviert. Dementsprechend sind bereits mehrere Methoden zur Stabilisierung solcher Lösungen bekannt. So wird beispielsweise gemäß EP-A 436744 der Einsatz von Oxidationsmitteln, insbesondere von Wasserstoffperoxid und Natriumperborat, in Mengen von 0,1 bis 3 Gew% bezogen auf die gesamte Lösung vorgeschlagen. Nachteilig daran ist die eigene relative Instabilität von z.B. Wasserstoffperoxid sowie die vielfältigen Reaktionsmöglichkeiten mit den auszurüstenden Systemen, zumindest bei den notwendigen Mengen. So können insbesondere Oxidationsmittel in den genannten Mengen nicht zur Konservierung von Materialien für die Photoindustrie, wie beispielsweise Gelatine, Photoemulsionen, Entwicklerbädern usw., eingesetzt werden. Isothiazolinone werden zudem von Peroxiden am Schwefel oxidiert und bilden Produkte mit unbekannter Toxizität.

Aus US 3,870,795 und der darauf basierenden US 4,067,878 ist bekannt, zur Stabilisierung Metallnitrate oder -nitrite, beispielsweise auch Silbernitrat, in Mengen von 1 bis 30 Gew.-% bezogen auf die gesamte Lösung zu verwenden. Andere Metallsalze außer Nitraten und Nitriten sind nach diesem Dokument unwirksam. Nachteilig an der Verwendung von Nitraten oder Nitriten ist die mögliche Bildung toxischer Nitrosamine, sowie die Gefahr, daß Bestandteile der Formulierung nitrosiert werden.

Darüber hinaus führt die Anwendung hoher Salzanteile in beispielsweise Wachsemulsionen oder Kunststoffdispersionen und daraus hergestellten Produkten, wie Farben, Klebstoffe oder Kunstharzputze, zu Unverträglichkeiten wie Koagulation oder Stippenbildung.

Es besteht daher Bedarf an einer Stabilisierungsmethode, die die geschilderten Nachteile nicht aufweist, breit einsetzbar und insbesondere für Materialien der Photoindustrie geeignet ist.

Es wurde nun überraschenderweise gefunden, daß die Aufgabe durch kleine Mengen an Edelmetallionen gelöst werden kann.

Die vorliegende Erfindung betrifft somit eine lagerstabile wäßrige Lösung eines oder mehrerer Isothiazolin-3-one der allgemeinen Formel I 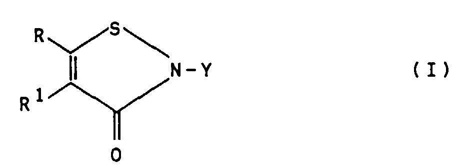 worin
- Y: Wasserstoff, (C₁-C₈)-Alkyl, (C₂-C₁₈)-Alkenyl, (C₃-C₁₂)-Cycloalkyl oder (C₆-C₁₀)-Aryl bedeutet,
und
- R und R¹: unabhängig voneinander Wasserstoff, Halogen, (C₁-C₄)-Alkyl oder C₄-Alkenyl bedeuten oder zusammen mit den sie tragenden Kohlenstoffatomen einen 3- bis 6-gliedrigen Kohlenstoffring bilden, der 1, 2 oder 3 Doppelbindungen enthält;
dadurch gekennzeichnet, daß sie ein Edelmetallion in einer Menge von bis zu 1 g/kg Verbindung der allgemeinen Formel I enthält.
Für Y stehendes (C₁-C₈)-Alkyl sowie für R oder R¹ stehendes (C₁-C₄)-Alkyl kann geradkettig oder verzweigt sein und beispielsweise Methyl, Ethyl, n-Propyl, i-Propyl, n-Butyl, i-Butyl, tert.-Butyl oder sek.-Butyl bedeuten. (C₁-C₈)-Alkyl kann auch noch beispielsweise für n-Pentyl, n-Hexyl, n-Heptyl oder n-Octyl stehen.

Auch (C₂-C₁₈)-Alkenylreste können geradkettig oder verzweigt sein und bedeuten insbesondere Vinyl oder Allyl. (C₃-C₁₂)-Cycloalkylreste sind insbesondere Cyclopentyl, Cyclohexyl und Cyclooctyl.
(C₆-C₁₀)-Akyl ist insbesondere Phenyl.

Halogen ist insbesondere Fluor, Chlor oder Brom. Der von R und R¹ zusammen mit den sie tragenden Kohlenstoffatomen gebildete Kohlenstoffring ist bevorzugt fünfgliedrig. Besonders bevorzugt ist er fünfgliedrig und weist eine Doppelbindung auf, d. h. ist ein annellierter Cyclopentenylring.

Y bedeutet bevorzugt Wasserstoff oder (C₁-C₈)-Alkyl und besonders bevorzugt Methyl.

R und R¹ bedeuten bevorzugt unabhängig voneinander Wasserstoff oder Halogen. Besonders bevorzugt bedeuten R Chlor und R¹ Wasserstoff.

Unter Edelmetallionen im Sinne vorliegender Erfindung werden insbesondere Ionen des Rutheniums, Rhodiums, Palladiums, Osmiums, Iridiums, Platins, Silbers und Golds verstanden. Silber-, Gold- und Palladiumionen sind bevorzugt. Die erfindungsgemäßen Lösungen enthalten die Edelmetallionen in Mengen von 0,01 mg bis 1 g/kg Verbindung der allgemeinen Formel I. Besonders bevorzugte Mengen sind 10 mg bis 200 mg/kg Verbindung der allgemeinen Formel I.

Dabei ist selbstverständlich zu beachten, daß die Obergrenze durch die Löslichkeit des eingesetzten Edelmetallsalzes bestimmt wird. Beispielsweise genügen aber im Falle von Ag^{⊕} Konzentrationen, die weit unterhalb der Löslichkeitsprodukte der schwerlöslichen Silberhalogenide liegen.

Die Edelmetallionen werden bevorzugt in Form ihrer Salze in die erfindungsgemaßen Lösungen eingebracht. Dabei ist die Art des Anions unkritisch, solange die Löslichkeit des Salzes zum Erreichen der notwendigen Edelmetallionkonzentration ausreicht. Geeignete Anionen sind beispielsweise Chlorid, Perchlorat, Bromid, Iodid, Nitrat, Sulfat, Acetat, Citrat, Ascorbat, Maleat, Oxalat, Methylsulfonat, Carbonat und Phosphat.

Die erfindungsgemäßen Lösungen enthalten die Verbindungen der allgemeinen Formel I in der Regel in Mengen von 0,1 bis 50Gew%, bezogen auf die gesamte Lösung. Bevorzugt ist ein Gehalt von 0,5 bis 10 Gew%. Auch hier wird die Obergrenze durch die Löslichkeit der Verbindungen der allgemeinen Formel I bestimmt.

Der Gehalt der erfindungsgemäßen Lösungen an Edelmetallionen bezogen auf die gesamte Lösung beträgt somit beispielsweise bei einem 50Gew%igen Gehalt an Verbindung der allgemeinen Formel I und einem Einsatz von 1 g/kg 0,05Gew%.

Die Verbindungen der allgemeinen Formel I können als solche, aber auch in Form ihrer bekannten Metallsalzkomplexe eingesetzt werden. Letztere sind beispielsweise in EP-A 436744 und US 3,870,795 beschrieben.

Die erfindungsgemäßen wäßrigen Lösungen können eine oder mehrere der Verbindungen der allgemeinen Formel I enthalten. Enthalten sie mehrere, so sind deren Mischungsverhältnisse untereinander vollkommen unkritisch. Bevorzugt beträgt das Verhältnis zweier Verbindungen der allgemeinen Formel I 1 : (0,1 bis 50), besonders bevorzugt 1 : (1 bis 15).
Eine besonders bevorzugte erfindungsgemäße wäßrige Lösung enthält 5-Chlor-2-methylisothiazolin-3-on und 2-Methylisothiazolin-3-on im Verhältnis (1 bis 10) : 1, insbesondere (2 bis 4) : 1.

Die erfindungsgemäßen wäßrigen Lösungen können neben den Edelmetallionen bzw. -salzen, produktionsbedingt oder aus sonstigen Gründen auch noch weitere lösliche Metallsalze enthalten. Als Kationen können dabei beispielsweise Lithium-, Natrium-, Kalium-, Calcium-, Barium-, Aluminium-, Eisen-, Mangan-, Nickel-, Kobalt-, Zinn- oder Zinkionen vorhanden sein. Mögliche Anionen sind Chlorid, Perchlorat, Bromid, Iodid, Nitrat, Sulfat, Acetat, Citrat, Ascorbat, Maleat, Oxalat, Methylsulfonat, Carbonat und Phosphat.

Die Konzentrationen solcher Salze sind unkritisch, solange kein Löslichkeitsprodukt überschritten wird und alle Ionen in Lösung bleiben.

Neben Wasser können die erfindungsgemäßen wäßrigen Lösungen auch noch wassermischbare organische Lösemittel enthalten. Geeignete wassermischbare organische Lösemittel sind beispielsweise Alkohole, wie Methanol, Ethanol, n-Propanol, i-Propanol und tert.-Butanol, Ketone, wie Aceton und Methylethylketon, Ether, wie Methoxybutanol, aber auch Glykole, sowie Di- und Polyglykole und deren Ether und Ester.

Diese organischen Lösemittel können in Mengen von 1 bis 50 Gew% bezogen auf die gesamte Lösung vorhanden sein. Schließlich können die erfindungsgemäßen Lösungen auch noch andere verträgliche Wirkstoffe enthalten, mit denen sich die mikrobiozide Effektivität der Verbindungen der allgemeinen Formel I steigern läßt. Geeignete Wirkstoffe sind insbesondere Bronopol, Chloracetamid, sowie Aldehyde, wie beispielsweise Formaldehyd und die daraus hergestellten Formaldehyddepotverbindungen, die in Mengen von 1 bis 20 Gew%, bezogen auf die gesamte Lösung eingesetzt werden können.

Die erfindungsgemäßen wäßrigen Lösungen werden in der Regel durch einfaches Lösen der Komponenten in Wasser hergestellt. Da die Verbindungen der allgemeinen Formel I normalerweise herstellungsbedingt als Hydrochloride vorliegen, muß nach deren Auflösen mittels Metallhydroxiden oder Ionenaustauschern neutralisiert werden.

Die Verbindungen der allgemeinen Formel I sowie ihre Metallsalzkomplexe sind bekannt und/oder können nach bekannten Methoden hergestellt werden. Beispielsweise sind aber auch konzentrierte Mischungen von 5-Chlor-2-methyl-isothiazolin-3-on und 2-Methylisothiazolin-3-on im Handel erhältlich.

Die erfindungsgemäßen wäßrigen Lösungen sind ausreichend stabil und weisen die Nachteile des Standes der Technik nicht auf. Sie können in allen technischen Systemen, in denen Mikroorganismen wachsen können, eingesetzt werden, z.B. in Kunststoffdispersionen, Dispersionsfarben, Metallbearbeitungsflüssigkeiten, Kühlkreisläufen, bei der Papierherstellung, in Treibstoffen usw.. Besonders vorteilhaft lassen sich die mit Silberionen stabilisierten erfindungsgemäßen Lösungen in der Photoindustrie wie z.B. in Photoemulsionen, in photografisch genutzten Bädern und ähnlichem einsetzen, bei denen der Zusatz anderer Metallsalze nicht möglich ist. Ebenso besonders vorteilhaft ist der Einsatz in Kunststoffdispersionen, die bei Zugabe der mit mehrwertigen Salzen wie z.B. Magnesiumnitrat oder Calciumnitrat stabilisierten Konservierungsmittel koagulieren.

Mikroorganismen, die sich mit den erfindungsgemäßen Lösungen besonders vorteilhaft bekämpfen lassen, sind Bakterien, wie z.B. Bacillus subtilis, Enterobacter aerogenes, Escherichia coli, Proteus vulgaris, Pseudomonas aeruginosa und Pseudomonas fluorescens; Hefen, wie z.B. Candida albicans und Saccharomyces cerevisiae; Schimmelpilze, wie z.B. Aspergillus niger, Chaetomium globosum, Penicillium funiculosum und Ulocladium consortiale; sowie Algen, wie z.B. Chlorella fusca und Anabaena cylindrica.

### Beispiel 1

Aus jeweils 10 g einer im Handel erhältlichen Mischung von 5-Chlor-2-methylisothiazolin-3-on (5CLMIT) und 2-Methylisothiazolin-3-on (MIT) mit einem Gewichtsverhältnis 5CLMIT : MIT von 8 : 1 und 90 g entionisiertem Wasser wurden stabilisierte Lösungen hergestellt (Lösung A : Ag-Gehalt 5,3 mg/ kg; Lösung B : Ag-Gehalt 0,53 mg/kg). Mit Essigsäure wurde ein pH-Wert von 4-5 eingestellt und die Lösungen bei 40°C in verschlossenen Glasgefäßen gelagert. Die Stabilität wurde über den Gehalt an 5CLMIT bestimmt, der nach 2, 4 und 15 Wochen mittels HPLC (250 mm lange RP-18 Säule vom Innendurchmesser 4 mm; Füllmaterial Hypersil ODS 5 µ; Gradientenelution mit Acetonitril/Wasser vom Verhältnis 20 : 80 auf 100 : 0 linear ansteigend; UV-Detektion bei 275 nm) gemessen wurde. Zum Vergleich diente eine unstabilisierte Lösung (Lösung C), sowie eine gemäß US 3,870,795 mit Cu(NO₃)₂ · 3H₂O stabilisierte Lösung (Lösung D).

| Ergebnisse: | | | | |
|---|---|---|---|---|
| Lösung | Lagerung | Gehalt 5CLMIT | Verlust 5CLMIT | Aussehen |
| A | | | | |
| | 0 | 1,12% | - | klar, farblos |
| 5,3 mg/kg Ag | 2 Wochen | 1,12% | 0% | " |
| | 4 " | 1,12% | 0% | " |
| | 15 " | 1,12% | 0% | " |

| B | | | | |
|---|---|---|---|---|
| 0,53 mg/kg Ag | 0 | 1,12% | - | klar, farblos |
| | 2 Wochen | 1,12% | 0% | " |
| | 4 " | 1,12% | 0% | " |
| | 15 " | 1,08% | 4% | " |

| C | | | | |
|---|---|---|---|---|
| ohne Stabilisator | 0 | 1,12% | - | klar, farblos |
| | 2 Wochen | 0,87% | 22% | trüb |
| | 4 " | 0,73% | 35% | trüb,Bodensatz |
| | 15 " | 0% | 100% | " |

| D | | | | |
|---|---|---|---|---|
| 8030 mg/kg Cu | 0 | 1,14% | - | klar,blau |
| | 2 Wochen | 1,14% | 0% | klar,blau |
| | 4 Wochen | 1,13% | 1% | schwach,trüb |

### Beispiel 2

Beispiel 1 wurde mit PdSO₄ als Stabilisator wiederholt (Gehalt an Pd: 8,5 mg/kg). Der ursprüngliche Gehalt an 5CLMIT von 1,14% war nach zwei Wochen unverändert und betrug nach 4 Wochen 1,09% (Verlust: 5%).

### Beispiel 3

Gemäß Beispiel 1 hergestellte Lösungen wurden mit Tetrachloroaurat (III) (HAuCl₄; Normallösung 0,1 g Gold FIXANAL® der Firma Riedel-de Haen AG, Seelze) stabilisiert und bei 50°C gelagert.

| Ergebnisse | | | |
|---|---|---|---|
| Lösung | Lagerung | Gehalt 5CLMIT | Verlust 5CLMIT |
| A | | | |
| 5 mg/kg Au | 0 | 1,24% | - |
| | 1 Woche | 0,96% | 23% |
| | 2 Wochen | 0,85% | 31% |

| B | | | |
|---|---|---|---|
| 0,5 mg/kg Au | 0 | 1,24% | - |
| | 1 Woche | 0,87% | 30% |
| | 2 Wochen | 0% | 68% |

| C | | | |
|---|---|---|---|
| ohne Stabilisator | 0 | 1,24% | - |
| | 1 Woche | 0% | 100% |

### Beispiel 4

Eine käufliche wäßrige Lösung von 10 Gew% 5-Chlor-2-methylisothiazolin-3-on (5CLMIT) und 3,6 Gew% 2-Methylisothiazolin-3-on (MIT), die produktionsbedingt 7% Magnesiumchlorid und 11% Magnesiumnitrat enthielt, wurde mit deionisiertem Wasser auf den Faktor 10 verdünnt. Es wurde durch Zusatz von Silbernitrat stabilisiert (Lösung A) und bei 50°C gelagert. Der Gehalt an 5CLMIT wurde wie in Beispiel 1 angegeben verfolgt. Die Lösung ohne AgNO₃-Zusatz (Lösung 3) entspricht der Lehre der US 3,870,795.

| Ergebnisse | | | | |
|---|---|---|---|---|
| Lösung | Lagerung | Gehalt 5CLMIT | Verlust 5CLMIT | Aussehen |
| A | | | | |
| | 0 | 1,02% | - | klar |
| 4,8 mg/kg Ag | 2 Wochen | 1,02% | 0% | " |
| | 6 Wochen | 1,02% | 0% | " |

| B | | | | |
|---|---|---|---|---|
| Vergleich | 0 | 1,04% | - | klar |
| | 2 Wochen | 1,01% | 3% | " |
| | 6 Wochen | 0,97% | 7% | Bodensatz |

### Beispiel 5

Beim Einsatz von 5CLMIT-haltigen Konservierungsmitteln, die mit Magnesiumsalzen stabilisiert sind, in Kunststoffdispersionen kann ein sogenannter Elektrolytschock eintreten, wobei das Polymer aus der Dispersion koaguliert und Stippen oder einen Bodensatz bildet.
Dazu wurde folgender Versuch gemacht:

0,5 g Lösung A gemäß Beispiel 1 wurden mit 100 g einer siebrückstandsfreien Reinacrylatdispersion homogen verrührt. Die Dispersion wurde 2 Tage bei 25°C gelagert, anschließend der Siebrückstand gemäß DIN 53 786 auf einem Drahtsiebboden mit einer Maschenweite von 0,056 mm bestimmt, weitere 3 Tage bis 50°C gelagert und schließlich die Siebanalyse wiederholt.
Zum Vergleich wurden 0,5 g einer Lösung gemäß US 3,870,795, enthaltend 1,1 Gew% Magnesiumnitrat und 0,7 Gew% Magnesiumchlorid und dem gleichen Wirkstoffgehalt (Lösung B) dem gleichen Verfahren unterworfen.

| Ergebnisse | | |
|---|---|---|
| Lösung | Menge der grobteiligen Anteile nach | |
| | 2 Tagen | 5 Tagen |
| A | | |
| 5,3 mg/kg Ag | 0 | 0 |

| B | | |
|---|---|---|
| 1,1% Mg(NO₃)₂ | 0,21 g | 0,25 g |
| 0,7% MgCl₂ | | |

## Patentansprüche

1. Lagerstabile wäßrige Lösung eines oder mehrerer Isothiazolin-3-one als eine Verbindung der allgemeinen Formel I 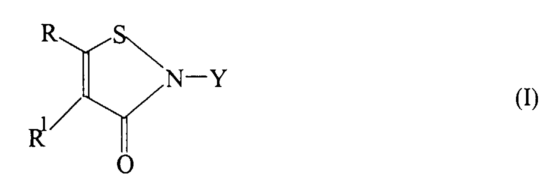 worin
Y Wasserstoff, (C₁-C₈)-Alkyl, (C₂-C₁₈)-Alkenyl,(C₃-C₁₂)-Cycloalkyl oder (C₆-C₁₀)-Aryl bedeuten, und
R und R¹ unabhängig voneinander Wasserstoff, Halogen, (C₁-C₄)-Alkyl oder C₄-Alkenyl bedeuten oder zusammen mit den sie tragenden kohlenstoffatomen einen 3-bis 6-gliedrigen Kohlenstoffring bilden, der 1, 2 oder 3 Doppelbindungen enthält;
dadurch gekennzeichnet, daß die Verbindung der allgemeinen Formel I in Mengen von 0,1 bis 50 Gew.-% auf die gesamte Lösung vorhanden ist und sie ein Edelmetallion in einer Menge von 0,01 mg/kg bis 1 g/kg der Verbindung der allgemeinen Formel I enthält.

2. Lösung gemäß Anspruch 1, dadurch gekennzeichnet, daß Y Wasserstoff oder (C₁-C₁₈)-Alkyl bedeutet.

3. Lösung gemäß Anspruch 1 oder 2, dadurch gekennzeichnet, daß R und R¹ unabhängig voneinander Wasserstoff oder Halogen bedeuten.

4. Lösung gemäß einem oder mehreren der Ansprüche 1 bis 3, dadurch gekennzeichnet, daß als Edelmetallion ein Ion des Rutheniums, Rhodiums, Palladiums, Osmiums, Iridiums, Platins, Silbers oder Golds eingesetzt wird.

5. Lösung gemäß einem oder mehreren der Ansprüche 1 bis 4, dadurch gekennzeichnet, daß die 5-Chlor-2-methyl-isothiazolin-3-on und 2-Methyisothiazolin-3-on im Verhältnis (1 bis 10):1 enthalten.

6. Verwendung einer Lösung gemäß einem oder mehreren der Ansprüche 1 bis 5 als mikrobiozides Mittel zum Einsatz in technischen Systemen, in denen Mikroorganismen wachsen können.

## Claims

1. A storage stable aqueous solution of one or more isothiazolin-3-one(s) as a compound represented by the general formula I 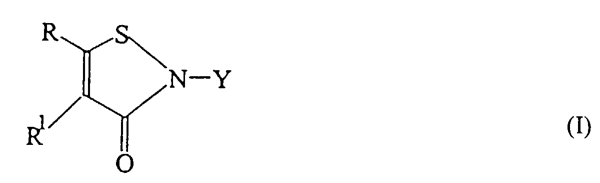 wherein
Y is hydrogen, (C₁-C₈) alkyl, (C₂-C₁₈) alkenyl, (C₃-C₁₂) cycloalkyl or (C₆-C₁₂) aryl; and
each of R und R¹ independently is hydrogen, halogen, (C₁-C₄) alkyl or C₄ alkenyl or, together with the carbon atoms to which they are bound form a 3 to 6 membered carbocyclic ring, containing 1, 2 oder 3 double bonds;
characterized in that the compound of the general formula 1 is present in an amount of 0,1 to 50 % by weight based on the total solution and contains a noble metal ion in an amount of 0,01 mg/kg to 1 g/kg of the compound of the general fomula 1.

2. The solution according to claim 1, characterized in that Y is hydrogen or (C₁-C₁₈) alkyl.

3. The solution according to claim 1 or 2, characterized in that each R and R¹ independently is hydrogen or halogen.

4. The solution according to any of the claims 1 to 3, characterized in that an ion of ruthenium, rhodium, palladium, osmium, iridium, platinum, silver or gold is used as noble metal ion.

5. The solution according to any of the claims 1 to 4, characterized in that it contains 5-chloro-2-methyl-isothiazolin-3-one and 2-methylisothiazolin-3-one in a ratio of (1 to 10) : 1.

6. Use of the solution according to any of the claims 1 to 5 as a microbiocide agent for the use in technical systems, wherein microorganisms may grow.

## Revendications

1. Solution aqueuse stable au stockage d'une ou plusieurs isothiazoline-3-ones, sous forme d'un composé de formule générale I 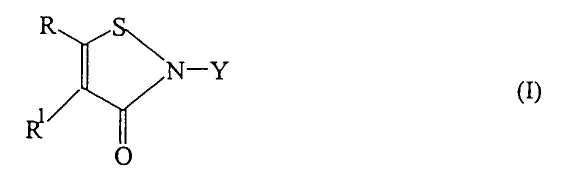 dans laquelle
Y est un atome d'hydrogène, un groupe alkyle en C₁-C₈, alcényle en C₂-C₁₈, cycloalkyle en C₃-C₁₂, ou aryle en C₆-C₁₀, et
R et R¹ représentent chacun indépendamment de l'autre un atome d'hydrogène ou d'halogène, ou un groupe alkyle en C₁-C₄ ou alcényle en C₄, ou encore, avec l'atome de carbone qui les porte, forment un noyau carboné à 3-6 chaînons, qui contient 1, 2 ou 3 doubles liaisons ;
caractérisée en ce que le composé de formule générale I est présent dans la totalité de la solution en des quantités de 0,1 à 50 % en poids, et qu'il contient un ion d'un métal précieux, en une quantité de 0,01 mg/kg à 1 g/kg du composé de formule générale I.

2. Solution selon la revendication 1, caractérisée en ce que Y est un hydrogène ou un groupe alkyle en C₁-C₁₈.

3. Solution selon la revendication 1 ou 2, caractérisée en ce que R et R¹ représentent chacun indépendamment de l'autre un atome d'hydrogène ou d'halogène.

4. Solution selon l'une ou plusieurs des revendications 1 à 3, caractérisée en ce qu'on utilise en tant qu'ion d'un métal précieux un ion du ruthénium, du rhodium, du palladium, de l'osmium, de l'iridium, du platine, de l'argent ou de l'or;

5. Solution selon l'une ou plusieurs des revendications 1 à 4, caractérisée en ce qu'elle contient de la 5-chloro-2-méthylisothiazoline-3-one et de la 2-méthylisothiazoline-3-one selon un rapport de (1 à 10):1.

6. Utilisation d'une solution selon l'une ou plusieurs des revendications 1 à 5 en tant qu'agent microbicide pour utilisation dans des systèmes techniques dans lesquels des microorganismes peuvent proliférer.
